# FASCICULE DE BREVET EUROPEEN

(11) **EP 2 538 215 B1**
(45) Date de publication et mention de la délivrance du brevet: **03.10.2018**
(21) Numéro de dépôt: 12184782.6
(22) Date de dépôt: 06.08.2009
(51) Int. Cl.: G01N 33/08

(54) **Procédé de mirage d'oeufs**
Verfahren zum Durchleuchten von Eiern
Egg candling method

(30) Priorité: 13.08.2008 FR 0855571
(43) Date de publication de la demande: 26.12.2012
(62) Demande divisionnaire de: 09781584.9
(73) Titulaire: Egg-Chick Automated Technologies, 35740 Pace (FR)
(72) Inventeur: Adjanohoun, Ephrem, 35740 Pace (FR)
(74) Mandataire: Novagraaf Technologies

(56) Documents cités:
- FR-A- 2 912 217
- JP-A- 2004 101 204
- US-A- 5 745 228

## Description

La présente invention concerne un procédé de mirage d'oeufs pour déterminer un état desdits oeufs.

Dans l'industrie de la volaille, notamment la production de poussins, il est connu des procédés de mirage automatique d'oeufs (cf. par exemple le brevet américain US 5,745,228 A) utilisant la transparence de l'oeuf à la fin de la période d'incubation pour différencier deux catégories d'oeufs: les oeufs pleins opaques à la lumière comprenant non seulement les oeufs fécondés contenant un embryon complètement développé vivant ou mort, mais également les oeufs contaminés ou pourris, et les oeufs perméables à la lumière comprenant les oeufs non fécondés, dits oeufs clairs, mais aussi les oeufs peu développés contenant un embryon mort précocement. Ces procédés consistent à émettre un flux lumineux en direction d'un oeuf à mirer et à analyser ensuite le flux lumineux passé à travers l'oeuf pour en déterminer son état, fécondé ou non fécondé, en fonction du taux de flux lumineux absorbé par l'oeuf. Pour ce faire, le système comporte de manière générale des moyens d'émission d'un flux lumineux, des moyens de réception du flux lumineux passé à travers l'oeuf et des moyens de traitement informatique du flux lumineux reçu par les moyens de réception. Les moyens de traitement informatique sont alors capables de différencier grossièrement les oeufs en fonction du taux du flux lumineux absorbé par l'oeuf. A la fin de la période d'incubation, l'oeuf fécondé renfermant un embryon vivant normalement développé est opaque. Mais, c'est aussi le cas des oeufs renfermant un embryon mort tardivement ou des oeufs contaminés et pourris. Ces derniers ne sont donc pas facilement différentiables des oeufs normalement développés. Quant aux oeufs qui sont perméables à la lumière à la fin de la période d'incubation, ils correspondent soit aux oeufs infertiles, soit aux oeufs renfermant un embryon mort précocement, sans qu'il soit possible de les distinguer de manière fiable.

Ce procédé de mirage par transparence ne permet donc pas de différencier avec certitude les oeufs vivants des oeufs morts à la fin de la période d'incubation. Il n'est pas non plus adapté pour être utilisé à un stade plus précoce du cycle de développement de l'oeuf, par exemple entre le 3^{ème} et le 17^{ème} jour du cycle d'incubation de l'oeuf de poule. Il ne permet pas de connaître avec certitude l'état fécondé ou non fécondé de l'oeuf en début de cycle du fait de la taille insignifiante de l'embryon. Il ne permet pas non plus de fournir des informations plus précises sur l'état vivant ou mort de l'embryon à un stade intermédiaire du cycle d'incubation et/ou sur son état de développement (développement normal ou pas de l'embryon à ce même stade intermédiaire du cycle d'incubation). Par ailleurs, il n'est pas non plus adapté pour déterminer un état retourné ou non retourné de l'oeuf en fonction de la position de sa chambre à air. Enfin, il ne donne aucune information sur l'intégrité de la coquille de l'oeuf, à savoir la présence ou non de fêlures sur la coquille.

De façon alternative, un mirage effectué manuellement à l'aide d'une source de lumière blanche ne permet pas non plus de pallier les inconvénients précités en raison de la visibilité médiocre des structures internes de l'oeuf. La demande de brevet japonaise JP 2004-101204 divulgue l'éclairement d'un oeuf avec une lumière a priori blanche, la capture d'une image de l'oeuf éclairé avec une caméra CCD couleur et une analyse de l'image capturée pour déterminer un état de développement de l'embryon en fonction de la présence, de l'absence ou de la taille des vaisseaux sanguins de l'oeuf. La demande de brevet FR 2 912 217 A divulgue l'illumination d'un oeuf fécondé avec une lumière verte focalisée à l'intérieur de l'oeuf, la capture d'une image de l'oeuf éclairé et le traitement de l'image capturée pour déterminer, en fonction de la présence d'un réseau sanguin, si l'embryon de l'oeuf est vivant ou est mort. Le but de la présente invention est de proposer une solution palliant tout ou partie des inconvénients précités.

A cet effet, la présente invention a pour objet un procédé de mirage d'oeufs pour déterminer au moins un état d'au moins un oeuf fécondé contenant un embryon selon la revendication 1. Ce procédé permet de déduire si l'embryon est vivant ou mort ou si son niveau de vitalité est normal ou pas en fonction de son âge.

L'utilisation de cette lumière verte, avantageusement sous forme diffractée, permet de faire apparaître très distinctement sur la coquille de l'oeuf l'ombre des veines et des vaisseaux du réseau sanguin alimentant l'embryon de l'oeuf du fait qu'ils sont positionnés contre les parois et membranes internes de la coquille.

On utilise une lumière verte dont la longueur d'onde est comprise entre 510 et 570 nm, de préférence entre 530 nm et 550 nm. Cette longueur d'onde peut être utilisée entre le 3^{ème} et le 17^{ème} jour du cycle d'incubation.

Typiquement, cette lumière verte fait apparaître principalement 3 zones de haut en bas de l'oeuf: une première zone très lumineuse correspondant à la chambre à air, une deuxième zone de luminosité intermédiaire qui correspond à la zone de l'oeuf contenant essentiellement les fluides embryonnaires, le réseau sanguin alimentant l'embryon vivant ou mort et, en position basse de cette zone, une masse opaque correspondant à une partie du corps de l'embryon et enfin une troisième zone très sombre dans laquelle aucune structure n'est identifiable.

Pendant l'étape d'analyse de ladite image, on détecte dans l'image la présence ou non d'un réseau de lignes sombres entrecroisées représentant l'ombre portée du réseau sanguin et on en déduit, en fonction de la présence ou non de ces lignes sombres et de leur dimension (épaisseur et/ou longueur), le niveau de vitalité de l'embryon.

Selon une particularité de l'invention, l'étape a) comprend l'éclairement de la partie supérieure de l'oeuf, disposé de sorte que son axe de révolution soit sensiblement vertical, de préférence au moyen d'une source de lumière disposée au-dessus de l'oeuf sensiblement sur l'axe de révolution de l'oeuf, et la capture d'une image de la partie supérieure de l'oeuf éclairé par une caméra, disposée à proximité de la source de lumière, l'axe de prise de vue de la caméra formant un angle compris entre 0 et 70° avec un plan de référence perpendiculaire à l'axe de révolution de l'oeuf et situé à mi-distance entre l'extrémité aplatie et l'extrémité pointue de l'oeuf.

Avantageusement, on prévoit la capture d'au moins deux images de l'oeuf éclairé, par au moins deux caméras distinctes couvrant avantageusement les deux cotés de l'oeuf pour s'assurer que le réseau sanguin, s'il est présent, apparaisse visuellement sur au moins l'une des images capturées quelle que soit sa position dans l'oeuf. Dans ce cas, l'étape a) comprend l'éclairement de l'oeuf avec une lumière verte, la capture par une première caméra d'une image I1 de l'oeuf éclairé et la capture par une deuxième caméra d'une image I2 de l'oeuf éclairé, les première et deuxième caméras étant disposées en vis-à-vis de part et d'autre d'un plan médian de l'oeuf comprenant l'axe de révolution de l'oeuf. L'étape b) comprend alors une analyse des deux images I1 et I2 pour déterminer le niveau de vitalité de l'embryon de l'oeuf.

Selon un deuxième mode de réalisation, on prévoit d'exciter l'embryon contenu dans l'oeuf et de capturer une image de l'oeuf éclairé avant et après excitation pour déterminer si le réseau sanguin de l'oeuf a changé de position et en déduire si l'oeuf est vivant ou mort. En effet, si l'oeuf est vivant, il réagit au stimulus et la position de son réseau sanguin ainsi que celle de la masse opaque du corps de l'embryon s'en trouve modifiée. Dans ce mode de réalisation, l'étape a) comprend l'éclairement de l'oeuf avec une lumière verte, la capture d'une image I1 de l'oeuf éclairé par une caméra, l'excitation de l'embryon de l'oeuf par un stimulus et, après excitation, l'éclairement de l'oeuf avec ladite lumière verte et la capture d'une image I'1 de l'oeuf éclairé par la même caméra. L'étape b) du procédé de l'invention comprend une comparaison des deux images I1 et I'1 pour déterminer l'état vivant ou l'état mort de l'oeuf et/ou confirmer le niveau de vitalité de l'embryon. Si les images I1 et I'1 sont différentes, l'oeuf est dans un état vivant.

Ce mode de réalisation avec excitation de l'embryon peut être utilisé avec ou sans analyse préalable de la dimension des veines du réseau pour déterminer l'état vivant ou mort de l'oeuf.

Avantageusement, dans ce mode de réalisation, on capture au moins deux images par deux caméras distinctes avant l'excitation de l'embryon et au moins deux images par ces mêmes caméras après excitation pour s'assurer que le réseau sanguin, s'il est présent dans l'oeuf, soit visible sur au moins une des images avant excitation et après excitation. Dans ce cas, l'étape a) comprend l'éclairement de l'oeuf avec une lumière verte, la capture d'une image I1 de l'oeuf éclairé par une première caméra et la capture d'une image I2 de l'oeuf éclairé par une deuxième caméra, l'excitation de l'embryon de l'oeuf par un stimulus, et, après excitation, l'éclairement de l'oeuf avec ladite lumière verte, la capture d'une image I'1 de l'oeuf éclairé par la première caméra et la capture d'une image I'2 de l'oeuf éclairé par la deuxième caméra. L'étape b) du procédé de l'invention comprend une comparaison des deux images I1 et I'1 et/ou une comparaison des deux images I2 et I'2 pour déterminer l'état vivant ou l'état mort de l'oeuf et/ou confirmer le niveau de vitalité de l'embryon.

Pour l'étape d'excitation, le stimulus employé est une perturbation temporaire du milieu environnant l'embryon déclenchant ou amplifiant son mouvement dans l'oeuf. Il peut prendre par exemple la forme d'une onde lumineuse, sonore et/ou thermique ou d'une vibration provoquée par exemple par un choc ou tout autre type de stimulus provoquant un mouvement chez l'embryon. Le stimulus est par exemple un signal lumineux intense, et/ou thermique, tel qu'une lumière blanche ou un signal laser.

Selon un perfectionnement applicable à tous les modes de réalisation, l'étape b) comprend en outre une analyse de l'image ou des images pour déterminer la présence ou non de la chambre à air en partie supérieure de l'oeuf en déduire si l'oeuf est dans un état retourné ou un état non retourné et éventuellement si la chambre à air est décalée par rapport a l'axe vertical de l'oeuf.

Selon un perfectionnement applicable à tous les modes de réalisation, l'étape b) comprend en outre une analyse de l'image ou des images pour analyser l'intégrité de la coquille et repérer des fêlures laissant s'échapper la lumière de façon plus intense.

Selon l'invention, pour deux oeufs voisins, l'éclairement est effectué de manière alternée entre les deux oeufs, comme défini dans la revendication 1.

L'invention sera mieux comprise, et d'autres buts, détails, caractéristiques et avantages apparaîtront plus clairement au cours de la description explicative détaillée qui va suivre de deux modes de réalisation particuliers actuellement préférés de l'invention, en référence aux dessins schématiques annexés, sur lesquels :
- la figure 1 représente un schéma de principe illustrant le procédé de l'invention;
- la figure 2 représente un organigramme montrant les étapes du procédé de l'invention selon un premier mode de réalisation de l'invention;
- la figure 3 représente un organigramme montrant les étapes du procédé de l'invention selon un deuxième mode de réalisation de l'invention;
- la figure 4 représente une vue de côté, perpendiculaire à la direction d'avancement des plateaux, d'un dispositif apte à mettre en oeuvre le procédé de l'invention, dans lequel le support de la rampe des sources de lumière et de la rampe de caméras est en position relevée;
- la figure 5 représente une vue de côté, parallèle à la direction d'avancement des plateaux, du dispositif représenté à la figure 4, et
- la figure 6 représente une vue analogue à celle de la figure 4, dans lequel le support de la rampe de sources de lumière et de la rampe de caméras est en position de mirage.

Selon l'invention, l'oeuf est éclairé avec une lumière verte pour faire apparaître visuellement sur la coquille une ombre portée du réseau sanguin alimentant l'embryon de l'oeuf s'il est présent dans l'oeuf. Au moins une image de l'oeuf est capturée puis traitée pour déterminer, en fonction de la présence ou non de ce réseau sanguin et de sa taille, si l'oeuf est dans un état vivant ou dans un état mort.

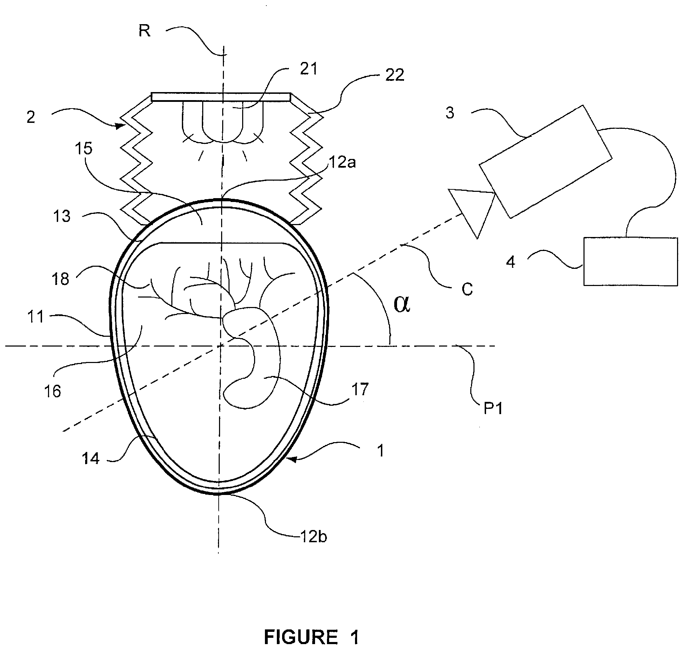

Le procédé de l'invention est illustré par la figure 1. L'oeuf miré est un oeuf fécondé 1 représenté de manière schématique. Il comprend une coquille 11 de forme ovoïde présentant une extrémité aplatie 12a et une extrémité pointue 12b. Une membrane coquillière externe très fine 13 tapisse l'intérieur de la coquille 11. Une membrane coquillière interne 14 également très fine tapisse partiellement la membrane coquillière externe 13 et s'en décolle au pôle apical de l'oeuf de manière à former une chambre à air 15 au niveau de l'extrémité aplatie 12a de l'oeuf. La chambre à air apparaît très lumineuse lorsqu'elle est éclairée par une lumière verte. La membrane coquillière interne 14 délimite une poche 16 renfermant, outre du blanc et des liquides embryonnaires transparents en partie supérieure et du jaune d'oeuf en partie inférieure, un embryon 17 en position intermédiaire au-dessus du jaune, lequel embryon est alimenté par un réseau sanguin 18 comprenant des veines et des vaisseaux capillaires. Le réseau sanguin est disposé majoritairement contre la membrane coquillière interne 14. Il est donc très proche de la coquille et baigne dans les liquides amniotiques transparents.

L'oeuf 1 est disposé de manière à ce que son axe de révolution, noté R, soit sensiblement vertical. Un plan de référence P1, perpendiculaire à l'axe de révolution R et coupant cet axe en un point situé à mi-distance entre l'extrémité aplatie 12a et l'extrémité pointue 12b de l'oeuf, est défini. La partie de l'oeuf 1 située au-dessus du plan de référence P1, appelée partie supérieure de l'oeuf, est éclairée par une source de lumière 2 disposée au-dessus de l'oeuf sensiblement sur l'axe de révolution R. La source de lumière 2 est une source de lumière verte dont la ou les longueurs d'onde sont comprises entre 510nm et 570nm. Cette lumière est particulièrement avantageuse pour faire apparaître les veines et vaisseaux du réseau sanguin 18 situés en périphérie de la face interne de la coquille. Cette lumière, qui est avantageusement diffractée, est absorbée par les éléments de l'oeuf transportant du sang. Lorsque l'oeuf est éclairé avec cette lumière verte, une ombre portée du réseau sanguin se dessine sur la coquille de l'oeuf. La présence ou non de ce réseau sanguin et la dimension (épaisseur et/ou longueur) des veines du réseau permettent de déterminer l'état vivant ou mort de l'embryon. La chambre à air apparait également très lumineuse.

La source de lumière 2 est par exemple formée par une ou plusieurs diodes électroluminescentes 21. La plage des longueurs d'onde de la lumière émise par la source 11 est comprise entre 510 nm et 570 nm. La lumière émise est soit monochromatique (1 seule longueur d'ondes dans cette plage) ou soit polychromatique (plusieurs longueurs d'onde dans cette plage). La lumière utilisée est avantageusement une lumière verte dont la longueur est comprise entre 530 nm et 550 nm.

La source de lumière 2 est avantageusement équipée d'une coupelle 22 de forme tubulaire à soufflet venant en appui sur l'extrémité aplatie 12a de l'oeuf lors de son éclairement. Cette coupelle forme un guide de lumière entre la source de lumière 2 et l'oeuf 1 et permet d'éviter les fuites de lumière ainsi que des entrées de lumière parasite. Cette coupelle est par exemple réalisée en élastomère noir.

Selon l'invention, une image de la partie supérieure de l'oeuf où apparaît l'ombre portée du réseau sanguin 18 sur la coquille est capturée par une caméra noir & blanc ou une caméra couleur 3 disposée à proximité de la partie supérieure de l'oeuf 1. L'axe de prise de vue, noté C, de la caméra 3 coupe de préférence l'axe de révolution R au niveau de son intersection avec le plan de référence P1. L'axe de prise de vue C forme un angle α, dit angle de latitude, avec le plan de référence P1. Cet angle est compris entre 0° et 70°. Si l'oeuf 1 est disposé dans un plateau comportant d'autres oeufs, les parois du plateau et les autres oeufs peuvent empêcher la capture de la partie supérieure de l'oeuf 1. Dans ce cas, on choisit un angle α dans la partie supérieure de la plage [0°,70°], par exemple un angle compris entre 30° et 70°. Une autre solution est de prévoir des moyens de levage de l'oeuf pour positionner la partie supérieure de l'oeuf dans le champ de l'objectif de la caméra.

Des moyens de traitement d'image 4 sont connectés à la caméra 3 pour traiter l'image capturée et déterminer, à partir de celle-ci, si l'embryon 17 est vivant ou mort.

Selon un premier mode de réalisation qui sera décrit en détail plus loin dans la description, le traitement effectué par les moyens de traitement d'image 4 consiste à déterminer si un réseau de lignes sombres correspondant au réseau sanguin de l'oeuf est présent dans l'image capturée, à évaluer l'épaisseur et/ou la longueur des veines et à comparer cette ou ces valeur(s) à une ou des valeurs de référence prédéterminées fonction de l'âge de l'oeuf. Si la valeur d'épaisseur évaluée et/ou la valeur de longueur évaluée est ou sont supérieure(s) à la valeur d'épaisseur de référence et/ou à la valeur de longueur de référence, l'oeuf est dans un état vivant. Sinon, il est dans un état mort.

Selon un second mode de réalisation également décrit en détail plus loin dans la description, l'embryon de l'oeuf est excité et une capture d'image est effectuée avant et après excitation de l'embryon. L'étape de traitement d'image du procédé consiste alors à comparer l'image avant excitation à l'image après excitation. Si l'oeuf est vivant, l'embryon réagit à l'excitation et son réseau sanguin ainsi que la masse sombre de l'embryon se déplace entre les deux images. Les deux images sont alors différentes. Si les deux images sont identiques, l'oeuf est mort.

Ces deux modes de réalisations peuvent être appliqués ensemble ou séparément. Ces deux modes de réalisation sont décrits de manière détaillée ci-après.

### Premier mode de réalisation

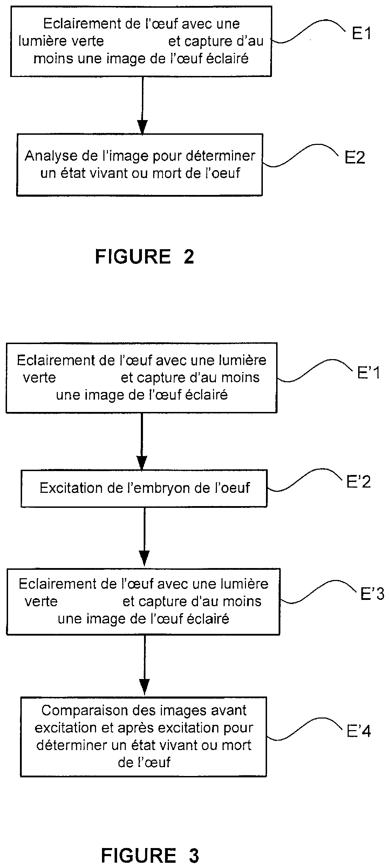

Selon ce premier mode de réalisation illustré par la figure 2, le procédé de l'invention comprend
- une étape E1 d'éclairement d'au moins une partie de l'oeuf à mirer avec une lumière verte, de préférence avec une longueur d'onde comprise entre 530nm et 550 nm, de manière à faire apparaître visuellement sur la coquille de l'oeuf une ombre portée du réseau sanguin de l'embryon si il est présent dans l'oeuf et de capture d'au moins une image dudit oeuf éclairé; et
- une étape E2 d'analyse de l'image capturée pour déterminer la présence ou non d'un réseau sanguin dans l'oeuf et en déduire un état vivant ou un état mort de l'oeuf.

L'étape E2 consiste à quantifier dans l'image l'épaisseur et/ou la longueur des lignes sombres représentant l'ombre du réseau sanguin de l'oeuf sur la coquille et à comparer ces valeurs à des valeurs de référence prédéterminées qui sont fonction de l'âge de l'oeuf. Pour un âge donné, si la valeur d'épaisseur est inférieure à la valeur d'épaisseur de référence prédéterminée pour cet âge et/ou si la valeur de longueur est inférieure à la valeur de longueur de référence prédéterminée pour cet âge, l'oeuf est considéré comme mort. Sinon, il est considéré comme vivant. Ces valeurs de référence augmentent de manière spécifique avec l'âge de l'oeuf.

Selon un perfectionnement, plusieurs valeurs d'épaisseur de référence et/ou plusieurs valeurs de longueur de référence sont définies pour chaque stade du cycle d'incubation de l'oeuf afin de caractériser, de manière plus fine, le niveau de vitalité de l'embryon à un stade donné du cycle d'incubation, la règle étant que, à un âge donné et en dessous d'un seuil donné, plus l'épaisseur et/ou la longueur des veines du réseau sanguin est faible, plus le niveau de vitalité de l'embryon est faible. Au-delà de ce seuil, l'embryon est considéré comme vivant et vigoureux. Ainsi, la dimension des veines évaluée à l'étape E2 est comparée à ces différentes valeurs de référence et le résultat de cette comparaison permet de définir un niveau de vitalité de l'embryon.

Selon un perfectionnement, au moins deux images de l'oeuf éclairé sont capturées par des caméras ayant des angles de prise de vue différents. Il arrive en effet que, suivant la position de l'embryon dans l'oeuf et suivant l'angle de prise de vue de la caméra 3, le réseau sanguin ne soit pas visible ou soit peu visible dans l'image capturée par la caméra 3. On prévoit donc avantageusement de prendre une autre image de l'oeuf éclairé par une autre caméra disposée par exemple à l'opposé de la caméra 3 par rapport au plan médian vertical de l'oeuf.

Selon ce perfectionnement, l'étape E1 comprend l'éclairement de l'oeuf avec une lumière verte et la capture d'une image I1 de l'oeuf éclairé par une première caméra, par exemple la caméra 3, et la capture d'une image I2 de l'oeuf éclairé par une deuxième caméra, non représentée sur la figure 1, lesdites première et deuxième caméras étant disposées symétriquement de part et d'autre de l'axe de révolution de l'oeuf. L'étape E2 comprend une analyse des deux images I1 et I2 pour déterminer la présence ou non d'un réseau sanguin et en déduire un état vivant ou un état mort de l'oeuf.

### Deuxième mode de réalisation

Selon ce deuxième mode de réalisation illustré par la figure 3, le procédé de l'invention comprend
- une étape E'1 d'éclairement de l'oeuf avec une lumière verte et de capture d'au moins une image I1 de l'oeuf éclairé par une caméra,
- une étape E'2 d'excitation de l'embryon de l'oeuf par un stimulus,
- une étape E'3 d'éclairement de l'oeuf avec ladite lumière verte et de capture, après excitation de l'embryon, d'une image I'1 de l'oeuf éclairé avec la même caméra, et
- une étape E'4 de comparaison des deux images I1 et I'1 pour en déduire l'état, vivant ou mort, de l'oeuf. Si l'embryon est vivant, le réseau sanguin et la masse opaque de l'embryon changent de position dans l'oeuf entre les images I1 et I'1. Si les images I1 et I'1 sont différentes, on peut donc en déduire que l'oeuf est dans un état vivant.

Le stimulus employé à l'étape E'2 est une perturbation temporaire du milieu environnant l'embryon. Il peut prendre la forme d'une onde lumineuse, sonore et/ou thermique ou d'une vibration provoquée par exemple par un choc ou tout autre type de stimulus provoquant un mouvement chez l'embryon. Le stimulus est par exemple un signal lumineux intense, et/ou thermique, tel qu'une lumière blanche ou un signal laser. Ce stimulus est de préférence un signal laser. Les moyens pour générer ce stimulus sont avantageusement prévus dans la source de lumière 2. Ces moyens sont par exemple une source de lumière blanche ou une diode laser disposée à coté des diodes électroluminescentes vertes de la source de lumière 2.

Selon un perfectionnement, au moins deux images de l'oeuf sont capturées sous des angles de prise de vue différents avant excitation et après excitation. Selon ce perfectionnement, l'étape E'1 comprend l'éclairement de l'oeuf avec une lumière verte, la capture d'une image I1 de l'oeuf éclairé par une première caméra et la capture d'une image I2 de l'oeuf éclairé par une deuxième caméra. L'étape E'2 d'excitation est inchangée. L'étape E'3 comprend l'éclairement de l'oeuf avec ladite lumière verte, la capture d'une image I'1 de l'oeuf éclairé par la première caméra et la capture d'une image I'2 de l'oeuf éclairé par la deuxième caméra. L'étape E'4 comprend alors une comparaison des deux images I1 et I'1 et/ou une comparaison des deux images I2 et I'2 pour déterminer l'état vivant ou l'état mort de l'oeuf. Si le réseau sanguin et la masse opaque de l'embryon ont changé de position entre les images I1 et I'1 ou entre les images I2 et I'2, l'oeuf est dans un état vivant.

En variante, l'étape E'4 comporte une analyse des deux images I1 et I2 et/ou des deux images I'1 et I'2 pour déterminer, à partir des lignes sombres qu'elles comportent, si l'oeuf est dans un état vivant ou mort, puis une comparaison des deux images I1 et I'1 et/ou des deux images I2 et I'2 pour valider l'état vivant ou mort déterminé par l'analyse.

Ce second mode de réalisation peut venir en complément du premier mode de réalisation pour confirmer le niveau de vitalité déterminé lors de la l'analyse de la dimension des veines du réseau sanguin.

Selon un perfectionnement applicable aux deux modes de réalisation, l'étape E2 ou E'4 comprend en outre une analyse d'au moins l'une des images I1, I2, I'1 et I'2 pour déterminer la position de la chambre à air 15 de l'oeuf et en déduire si l'oeuf est dans un état retourné ou un état non retourné. L'oeuf est défini comme étant dans un état retourné lorsque sa chambre à air 15 est localisée dans la partie inférieure de l'oeuf et comme étant dans un état non retourné lorsque sa chambre à air est localisée dans la partie supérieure de l'oeuf. La détection de cet état est importante pour l'injection de vaccins dans l'oeuf et/ou le décalotage de celui-ci. L'analyse de l'image consiste alors à détecter si la partie supérieure de l'oeuf comporte ou non une zone très lumineuse correspondant à la chambre à air de l'oeuf. Si une telle zone est détectée dans la partie supérieure de l'oeuf, celui-ci est dans un état non retourné. L'analyse d'image peut également permettre de voir si la chambre à air est décalée par rapport a l'axe vertical de l'oeuf.

Selon un autre perfectionnement applicable aux deux modes de réalisation, l'étape E2 ou E'4 comprend en outre une analyse d'au moins l'une des images I1, I2, I'1 et I'2 pour vérifier l'intégrité de la coquille de l'oeuf et détecter la présence éventuelle de fêlures. Ces fêlures ont pour caractéristique de laisser passer la lumière. La détection des fêlures revient alors à détecter dans les images capturées la présence de lignes lumineuses très intenses.

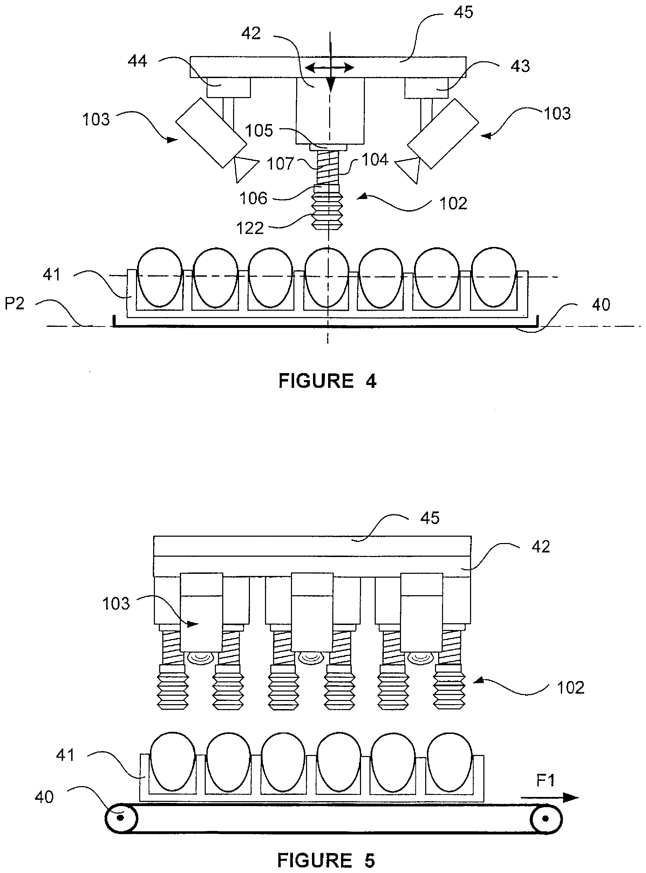

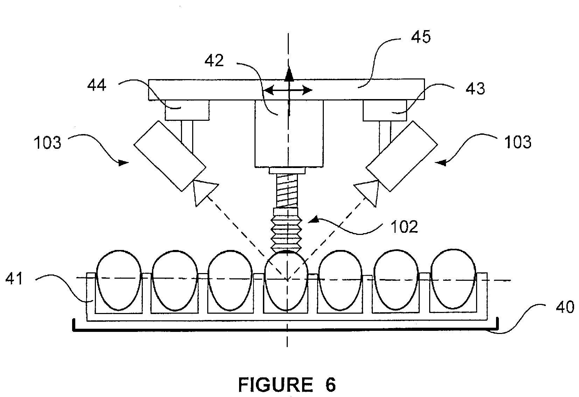

Les figures 4 à 6 représentent de manière schématique un dispositif de mirage automatique apte à mettre en oeuvre le procédé de l'invention. Les figures 4 et 5 représentent respectivement des vues de côté, respectivement transversale et parallèle à la direction d'avancement des plateaux, du dispositif en position relevée et la figure 6 représente une vue de côté analogue à celle de la figure 4 dans laquelle le dispositif est en position de mirage. Le dispositif comporte des moyens de convoyage 40 pour transporter des plateaux d'incubation d'oeufs 41, suivant un chemin de convoyage le long duquel sont disposés des moyens d'éclairement 102 et des moyens de capture d'image 103 des oeufs éclairés. Des moyens de traitement d'image, non représentés sur les figures 4 et 6, sont prévus pour traiter les images capturées par les moyens de capture d'image 103.

Les moyens de convoyage 40 comprennent par exemple un convoyeur de type à bande sans fin, définissant un plan de convoyage P2 (fig.4) de plateaux, apte à déplacer les plateaux 41 dans la direction d'avancement F1. Les plateaux comprennent par exemple des rangées longitudinales d'alvéoles, les rangées étant alignées ou décalées (alvéoles disposées en quinconce) entre elles. Dans l'exemple illustré, le plateau comprend 7 rangées alignées de 6 oeufs disposées parallèlement à direction d'avancement F1.

Les moyens d'éclairement 102 et les moyens de capture d'image 103 sont adaptés pour traiter les oeufs rangée par rangée. Les moyens d'éclairement 102 sont constitués d'une rangée de sources de lumière disposées côte à côte sur une rampe horizontale 42, dite d'éclairement. Dans l'exemple illustré, la rampe d'éclairement est placée parallèlement à la direction d'avancement F1 au dessus du chemin de convoyage. Une source de lumière 102 est prévue pour chaque oeuf d'une même rangée. Chaque source de lumière 102 comprend un tube 104 comprenant à son extrémité supérieure une embase 105 pour fixer le tube 104 à la rampe 42 et portant à son extrémité inférieure une ou plusieurs diodes électroluminescentes montées sur une douille 106. La douille 106 est montée coulissante sur le tube 104 et est sollicitée élastiquement par un ressort 107 monté entre l'embase 105 et la douille 106. La douille 106 est de préférence équipée d'une coupelle 122 en forme de soufflet pour guider la lumière produite par la ou les diodes vers l'oeuf et venir en contact avec la partie supérieure de l'oeuf lorsque la rampe d'éclairement 42 est abaissée (position de mirage).

Les moyens de capture d'image 103 sont constitués d'une pluralité de caméras disposées sur au moins une rampe horizontale 43. La rampe 43 est placée parallèlement à la direction d'avancement F1 au dessus du chemin de convoyage. Une caméra de la rampe est prévue pour chaque oeuf ou pour chaque groupe d'oeufs de la rangée disposés côte à côte. Dans l'exemple illustré, une caméra est prévue pour deux oeufs. Avantageusement, une deuxième rampe horizontale 44 munie de caméras est disposée symétriquement à la rampe 43 de caméras par rapport à la rampe de sources de lumière. Les caméras sont destinées à capturer une image de la partie supérieure des oeufs de la rangée.

Les rampes de caméras 43 et 44 et la rampe de sources de lumière 42 sont montées sur un support 45 apte à se déplacer verticalement entre une position relevée (position haute) et une position de mirage (position basse) et transversalement. Le déplacement du support 45 est commandé par des moyens de commande non représentés sur les figures 4 à 6. Ces moyens de commande commandent également l'allumage des sources de lumière et la capture d'images par les caméras. Il est à noter que ces moyens de commande peuvent être réunis avec les moyens de traitement d'image 4 dans une unité de commande centrale du dispositif.

En fonctionnement, un plateau est acheminé, sous le support 45, ledit support étant en position relevée avec les sources de lumière de la rampe d'éclairement 42 positionnées au dessus d'une rangée d'oeuf du plateau. Les moyens de commande déplacent verticalement vers le bas le support jusqu'à ce que la coupelle des sources de lumière vienne en appui sur la partie supérieure des oeufs de la rangée. Le support est alors en position de mirage (position basse). Les sources de lumière sont alors allumées. Les caméras disposées symétriquement de part et d'autre des sources de lumière capturent alors, successivement ou simultanément, des images des oeufs éclairés. Les moyens de commande déplacent ensuite verticalement vers le haut le support 45 jusqu'à sa position relevée (position haute) puis transversalement pour positionner les sources de lumière au-dessus de la rangée suivante d'oeufs. Les images capturées par les caméras sont transmises aux moyens de traitement d'image au fur et à mesure de leur capture ou après capture des images de l'ensemble des oeufs du plateau. Lorsque toutes les rangées du plateau ont été traitées, le convoyeur est actionné pour acheminer un nouveau plateau à traiter.

Dans l'exemple illustré, une seule caméra est prévue pour chaque paire d'oeufs voisins de la rangée. On prévoit alors un éclairement alternée entre les deux oeufs de la paire et une capture d'image séparée pour les deux oeufs. On éclaire d'abord un oeuf, on capture une image pour cet oeuf avec la caméra, puis on éclaire l'autre oeuf et on capture l'image pour l'autre oeuf. Ainsi, pour une rangée d'oeufs, tous les oeufs de rang pair de la rangée sont éclairés simultanément dans un premier temps puis tous les oeufs de rang impair sont éclairés simultanément dans un second temps.

Pour le traitement de plateaux décalés dans lesquels les oeufs de deux rangées successives sont disposés en quinconce, les moyens de déplacement sont en outre aptes à déplacer longitudinalement le support 45 pour passer d'une rangée à l'autre. Il est également possible de prévoir deux rampes de sources de lumière, montées sur un même support ou chacune sur un support, les sources de lumière des deux rampes étant décalées longitudinalement l'une par rapport à l'autre et chaque rampe de sources de lumière étant associée à une ou deux rampes de caméras.

Avantageusement, les sources de lumière sont munies de moyens permettant de faire varier la luminosité afin d'affiner la qualité des images prises par les caméras.

Avantageusement, plusieurs images peuvent être prises dans la même position par une même caméra avec pour seule différence entre elles soit l'intensité lumineuse des sources lumineuses, soit la durée d'exposition utilisée pour la prise d'image, afin d'affiner la qualité des détails pour l'analyse du réseau sanguin et/ou d'autres éléments accessoires contenus dans l'oeuf.

Pour la mise en oeuvre du second mode de réalisation du procédé de l'invention (avec excitation de l'embryon), les sources de lumière la rampe d'éclairement sont équipés de moyens d'excitation tels qu'une diode laser.

Selon un perfectionnement, le dispositif est équipé de moyens de levage des oeufs pour faciliter la capture des images lorsque les oeufs sont serrés, par exemple lorsqu'ils sont disposés en quinconce dans les plateaux d'incubation, ou lorsque la taille des oeufs environnant l'oeuf à analyser gène la prise d'image, par exemple lorsqu'un oeuf très gros est disposé devant l'oeuf à analyser de plus petite taille.

Bien que l'invention ait été décrite en liaison avec différents modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention comme défini par la revendication ci-jointe. On peut prévoir de traiter plusieurs rangées d'oeufs à la fois en utilisant plus de rampes de sources de lumière et rampes de caméras.

## Revendications

1. Procédé de mirage d'oeufs pour déterminer au moins un état d'au moins un oeuf fécondé contenant un embryon, ledit au moins un oeuf fécondé étant disposé dans un plateau (41) comprenant une pluralité d'alvéoles destinées à recevoir une pluralité d'oeufs, lesdites alvéoles étant organisées en rangées disposées parallèlement à la direction d'avancement dudit plateau **caractérisé en ce qu'**il comporte les étapes suivantes:
a) éclairement dudit oeuf avec une lumière verte ayant une ou plusieurs longueurs d'onde comprises entre 510nm et 570 nm de préférence entre 530 nm et 550 nm et capture d'au moins une image dudit oeuf éclairé; et
b) analyse de ladite image pour déterminer, en fonction de la présence ou non d'un réseau sanguin alimentant l'embryon dudit oeuf dans ladite image et, en cas de présence d'un réseau sanguin, de la dimension des veines dudit réseau sanguin, un niveau de vitalité de l'embryon dudit oeuf;
et **en ce que**, pour une rangée d'oeufs, on éclaire simultanément tous les oeufs de rang pair dans un premier temps puis, dans un second temps, on éclaire simultanément tous les oeufs de rang impair.

## Patentansprüche

1. Verfahren zum Durchleuchten von Eiern zum Bestimmen zumindest eines Zustandes von zumindest einem befruchteten Ei, das einen Embryo enthält, wobei das zumindest eine befruchtete Ei auf einem Tablett (41) platziert wird, das eine Vielzahl von Zellen umfasst, die zur Aufnahme einer Vielzahl von Eiern bestimmt sind, wobei die Zellen in Reihen organisiert sind, die parallel zur Fortbewegungsrichtung des Tabletts angeordnet sind, **dadurch gekennzeichnet, dass** es die folgenden Schritte umfasst:
a) Beleuchten des Eis mit einem grünen Licht, das eine oder mehrere Wellenlängen zwischen 510 nm und 570 nm, vorzugsweise zwischen 530 nm und 550 nm, aufweist, und Aufzeichnen von zumindest einem Bild des beleuchteten Eis; und
b) Analysieren des Bildes zum Bestimmen, in Abhängigkeit vom Vorhandensein oder nicht eines Blutgefäßnetzes zur Versorgung des Embryos des Eis auf dem Bild, und im Falle des Vorhandenseins eines Blutgefäßnetzes, der Größe der Venen des Blutgefäßnetzes, eines Vitalitätsniveaus des Embryos des Eis;
und dadurch, dass für eine Reihe von Eiern alle Eier einer geradzahligen Reihe zuerst gleichzeitig beleuchtet werden, danach alle Eier einer ungeradzahligen Reihe gleichzeitig beleuchtet werden.

## Claims

1. Egg candling method for determining at least one state of at least one fertilised egg containing an embryo, said at least one fertilised egg being placed on a tray (41) comprising a plurality of cells for receiving a plurality of eggs, said cells being organised in rows arranged parallely to the direction of movement of said tray, **characterised in that** it includes the steps of:
a) illuminating said egg with a green light having one or more wavelengths lying in the range 510 nm to 570 nm, preferably in the range 530 nm to 550 nm, and capturing at least one image of said illuminated egg; and
b) analysing said image to determine a vitality level of the embryo of said egg based on the presence or absence of a network of blood vessels feeding the embryo of said egg in said image and, in the event of the presence of a network of blood vessels, based on the dimensions of the veins of said network of blood vessels,
and **in that**, for a row of eggs, all the even eggs of the row are simultaneously illuminated in a first time then all the uneven eggs are simultaneously illuminated in a second time.
